Europäisches Patentamt

European Patent Office

Office européen des brevets

(19)

(11) Publication number: **0 071 610**
**B1**

(12) **EUROPEAN PATENT SPECIFICATION**

(45) Date of publication of patent specification: **12.03.86**

(51) Int. Cl.⁴: **A 61 F 2/24**

(21) Application number: **82900160.1**

(22) Date of filing: **02.12.81**

(86) International application number:
**PCT/RO81/00009**

(87) International publication number:
**WO 82/02829 02.09.82 Gazette 82/21**

(54) **CARDIAC VALVULAR BIOPROSTHESIS AND THE METHOD OF MANUFACTURE THEREOF.**

(30) Priority: **04.02.81 RO 103294**

(43) Date of publication of application:
**16.02.83 Bulletin 83/07**

(45) Publication of the grant of the patent:
**12.03.86 Bulletin 86/11**

(84) Designated Contracting States:
**DE FR**

(56) References cited:
**FR-A-2 038 843**
**FR-A-2 355 492**
**FR-A-2 451 189**
**GB-A-1 253 442**
**GB-A-1 264 471**
**GB-A-1 264 472**
**US-A-3 570 014**
**US-A-3 755 823**
**US-A-3 983 581**
**US-A-4 035 849**
**US-A-4 106 129**
**US-A-4 172 295**

(73) Proprietor: **INSTITUTUL DE CERCETARI
VETERINARE SI BIOPREPARATE "PASTEUR"
Sos. Giulesti, no. 333
Bucharest (RO)**

(72) Inventor: **POP D.POPA, Ioan
Str.Muzeul Zambaccian Nr.29
Sector 1 cod.71277 Bucuresti (RO)**
Inventor: **GHERGHICEANU, Dan
Str.Ing.Cucu Starostescu Nr.10
Sector 1 cod.71277 Bucuresti (RO)**
Inventor: **DOBRE, Mircea
Str.Drumul Taberei Nr.109
Bl.A7, Sector 6 cod.77641, Bucuresti (RO)**

(74) Representative: **Reinhard, Skuhra, Weise
Leopoldstrasse 51
D-8000 München 40 (DE)**

Courier Press, Leamington Spa, England.

## Description

The present invention relates to a cardiac valvular bioprosthesis for use in cardiac valvular replacements and to a method of manufacture thereof.

Metal valvular prostheses of different construction are known. The drawback of these prostheses is that the patents have to be submitted to a chronic anticoagulant treatment.

Bioprostheses made from a pericardium of calf, human dura mater and broad fascia are known too. The drawbacks of these protheses reside in that their cusps do not present an adequate anatomic form and their durability is poor.

Valvular bioprostheses from an aorta of a domestic pig are also known. The drawback of these protheses is that they present a reduced durability due to the short fibres with great fibrilar spaces for depositing fat.

US—A—3 570 014 discloses a cardiac valvular bioprotheses comprising an aortic valve of biological material applied on a metal or plastic support with a linking stratum and a fixing ring.

According to the invention there is provided a cardiac valvular bioprosthesis, comprising an aortic valve of biological material applied on a metal or plastic support with a linking stratum and a fixing ring, having an aortic valve from a wild boar or a bear, and a linking stratum of knitted textile material, said material having an inner cylindrical member and an outer cylindrical member fixed to said support by means of said ring which is of textile material, a plastics material or silicone rubber.

The aortic valve of a wild boar is a valve with three deep cusps that form some sinuses which bulge out under the horizontal plane passing through the valvular ring. The cusps appear semitransparent, the maximum transparence being at the center of the cusps. The presence of the dense fascilolae of fibres is also observed along the cusps between commissures, approximately parallel to the free edges of the cusps, the length of these fibres and their almost parallel orientation with small spaces between the fibres giving to the tissue a durability and elasticity, if the tissue is submitted to a very intensive mechanical requirement.

The support for the valve is formed with an annular base and ·a superstructure with three arms disposed at 120° on said base and being connected by a curve similar to the natural biological valve, each arm having an essentially isosceles triangular cut out, each of said cut outs having a small side on the base and its apex opposite to the base being provided with a protruberance for fixing the material of the seaming. The diameter of the base depends on the diameter of the valve which must be replaced. On the circumference of the base there is provided a cut out of 1—2 mm width, interrupted by some remaining portions, three of which are placed in the middle of the apex and the other halfway between each pair of apices.

According to the invention, there is also provided a method of manufacture of the cardiac valvular bioprothesis, wherein an aortic valve, an aortic wall and part of the interventricular septum are taken immediately after butchering of an animal, washed with physiologic serum to remove blood, and the valve is applied on a metal or plastic support, previously dressed with a fabric member and being provided with a ring on the outside, comprises the steps of taking said aortic valve, aortic wall and said part of the ventricular septum from a wild boar or a bear, removing muscular fibres septal cusps and tissue surplus from the aorta, introducing swabs in the cusps, which are thereafter introduced in a preserving and sterilizing solution in containers at 5—12°, taking out the biological material after 48 hours and peeling off the aortic wall, separately dressing said support completely with a cylindrical knitted material by fixing an outer cylinder thereof to the support by means of applying said ring which is of textile material, a plastics material or silicone rubber, and by bending and sewing an inner cylinder thereof over the ring, fixing the prepared biological material with two seams to the support and applying a band of knitted material for protection of the seam.

Hence, the preparing consists in eliminating all muscular fibres from the surface of the cusps and cutting the aortic wall in accordance with the form of the superior edge of the metal support, dressed with a knitted material. The metal support is advantageously prepared separately by dressing with knitted textile material having a tubular form consisting of a inner cylindrical member and an outer cylindrical member. The inner cylinder is introduced from the bottom upwards through the metal support. The upper end of the outer cylinder is bent over the apex of the arms and is drawn out under the lower edge of the support, 8—10 mm shorter than the other end of the inner cylinder of the knitted textile material. Thereafter the surplus of textile material, included between the arms, is removed, and the edges are sewn with thread from the exterior to the interior of the edges for being joined together.

The exterior cylinder of the knitted material is fixed in a few points (six) on the base of the metal support. The surplus is cut and bent over its edge, the ring is applied and fixed in a few points on the support of the base through the textile stratum and the cut out in the base. The edge of the inner cylinder is bent over the ring and sewn around with continuous thread.

Thereafter the biological material, previously prepared on the dressed support, is mounted by sewing with two threads, its introduction being made through the interior of the support. A first seam is made between the aortic valvular ring and the inferior edge of the dressed metal support. The second seam is made between the prepared aortic wall and the superior edge of the support. Finally, a band from the same knitted textile material is applied on the superior edge of the dressed support and the biological material thus sewn so as to cover the seam between the biological material and the support for protecting the seam.

The whole method of manufacture is performed in a sterile medium, and the manufactured bioprothesis is kept in a preserving and sterilizing solution until its use.

The cardiac valvular prothesis and the method of manufacture thereof present the following advantages:

— a high durability;

— no requirements of permanent anticoagulant treatment; and

— the use of this prosthesis is indicated specially for certain categories of patents as young women, ulcer patients, patients having disorders of sanguine chrasa, special social circumstances (bradipsychics, difficult access to medical assistance), and hypertensive patients for whom the bioprostheses of a pig are inadvisable.

An example of a bioprosthesis according to the invention is shown in figures 1—4.

Figure 1 shows a schematic vertical seciton through a bioprosthesis according to the invention.

Figure 2 shows a schematic vertical section through an arm along line A—A in figure 4;

Figure 3 shows a schematic section along line B—B in figure 4.

Figure 4 shows a side view of the metal support.

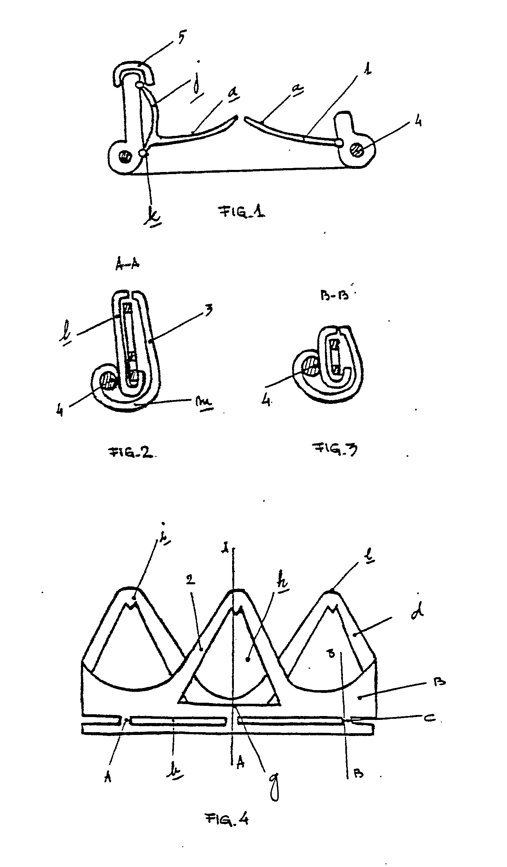

In accordance with the invention the cardiac valvular bioprothesis 1 consists of an aortic valve of a wild boar or a bear. This aortic valve has a special structure and is applied on a metal of plastic support 2 by means of a linking annular stratum 3 of knitted textile material. The fastening at the heart is made by means of a ring 4, whose folds can be made from knitted material, plastics material or silicone rubber.

The aortic valve of a wild boar has three deep cusps *a* formed from dense fascicolae of fibres along the cusps between commissures approximately parallel to the free edges thereof, the length of that fibres and their parallel orientation imparting a greater durability and elasticity to the tissue. The support 2 of the bioprosthesis 1 comprises a base A and a superstructure B. The base A has an annular form with a diameter depending on the diameter of the valve (between 15—33 mm), which must be replaced. On the circumference the base has a cut out *b* of 1—2 mm interrupted by some remaining portions *c*, three of which are placed in the middle of the base of the arms *d* and the other three halfway between the arms *d*.

The superstructure B is formed by three arms *d* of an essentially isosceles triangular form, disposed at 120° between superior rounded apices *e*. Adjacent apices *e* are connected by a curve *f* which faithfully follows the natural form of the biological valve, which is to be mounted on the support.

Each arm *d* has an essentially isosceles triangular cut out *h* a small side *g* being on the base A and the apex opposite to the base being rounded and being provided with a protuberance *e* for fixing the material of the seaming.

The method of manufacture of the cardiac valvular bioprosthesis consists in taking the aortic valve, the aortic wall and a part of the ventricular septum *k* from a wild boar or a bear, washing the biological material in physiological serum to remove the blood, removing the muscular fibres from the septal cusps and the tissue surplus from the aorta. Then swabs are introduced in the cusps in order to maintain the form. The biological material is then introduced in a preparing and sterilizing solution of glutaraldehyde 0.625 tamponed or provided with phosphat at pH 7.2—7.4.

The biological material is deposited in containers at 5—10°, and is taken out after 48 hr and prepared for its mounting on the support 2.

The preparation consists in removing the muscular fibres from the surface of the cusps *a* and in cutting the aortic wall *j* in accordance with the superior edge of the support 2, dressed with a stratum of knitted textile material. The metal support 2 is prepared separately by dressing it with knitted textile material of tubular form, consisting of an inner and an outer cylinder. The inner cylinder is introduced from the bottom upwards through the support 2.

The stratum 3 is bent over the apices *e*, and the outer cylinder 1 is pulled under the inferior edge of the support 2, 8—10 mm shorter than the end *m* of the stratum 3. Thereafter the material surplus included between the arms *d* is removed and the edges are sewn with thread from the exterior to the interior of the edges for being affixed.

The outer cylinder 1 is fixed at a few points (six) on the base A, the surplus is cut and it is bent over the edges of the support. The ring 4 is applied and fixed at a few points on the base A. The end *m* of the inner cylinder is bent over the ring 4 and is sewn around with a continuous thread.

Thereafter the previously prepared biologic material is mounted on the support by sewing with two threads, its introduction being made through the interior of the support 2.

A first seam is made between the aortic valvular ring and the inferior edge of support 2, and the second seam is made between the prepared aortic wall *j* and the superior edge of support 2. Then, a band 5 of the same textile material is applied on the superior edge of dressed support 2 and the sewn aortic wall *j* so that it covers the seam between the aortic wall *j* and support 2 for protection.

The whole process of manufacture is performed in a sterile medium. Until its use the prepared bioprosthesis is kept in a preserving and sterilizing solution.

The cardiac valvular bioprosthesis according to the invention can be manufactured without problems, since the biological material and the other means for preparing the bioprosthesis are available. For human use of the prosthesis it is necessary to test them by some apparatuses which determine their resistance and their hydrodynamic performance.

## Claims

1. Cardiac valvular bioprosthesis, comprising an aortic valve of biological material (1) applied on a metal or plastic support (2) with a linking stratum (3) and a fixing ring (4) characterized by an aortic valve from a wild boar or a bear, and a linking stratum (3) of knitted textile material, said material having an inner cylindrical member and an outer cylindrical member (1) fixed to said support (2) by means of said ring (4) which is of textile material, a plastics material or silicone rubber.

2. Bioprosthesis, according to claim 1, characterized in that said support (2) is formed with an annular base (A) and a superstructure (B) with three arms (d) disposed at 120° on said base (A) and being connected by a curve (f) similar to the natural biological valve, each arm (d) having an essentially isosceles triangular cut out, each of said cut outs having a small side (g) on the base (A) and its apex opposite to the base (A) being provided with a protuberance (i) for fixing the material of the seaming.

3. Method of manufacture of the cardiac valvular bioprosthesis according to claim 1 or 2, wherein an aortic valve, an aortic wall and part of the interventricular septum are taken immediately after butchering of an animal, washed with physiologic serum to remove blood, and the valve is applied on a metal or plastic support, previously dressed with a fabric member and being provided with a ring on the outside, characterized by the following steps:

Taking said aortic valve, aortic wall and said part of the ventricular septum from a wild boar of a bear;

removing muscular fibres from septal cusps and tissue surplus from the aorta;

introducing swabs in the cusps, which are thereafter introduced in a preserving and sterilizing solution in containers at 5—12°;

taking out the biological material after 48 hours and peeling off the aortic wall,

separately dressing said support completely with a cylindrical knitted material by fixing an exterior cylinder thereof to the support by means of applying said ring, which is of textile material, a plastics material or silicone rubber, and by bending and sewing an interior cylinder thereof over the ring;

fixing the prepared biological material with two seams to the support; and

applying a band of knitted material for protection of the seam.

## Revendications

1. Bioprothèse valvulaire cardiaque comprenant une valvule aortique en une matière biologique (1) appliquée sur un support métallique ou plastique (2) avec une couche de liaison (3) et une bague de fixation (4), caractérisée par une valvule aortique provenant d'un sanglier ou d'un ours et par une couche de liaison (3) en une matière textile tricotée, ladite matière comprenant un élément cylindrique interne et un élément cylindrique externe (1) fixé sur ledit support (2) au moyen de ladite bague (4) qui est en matière textile, en matière plastique ou en caoutchouc au silicone.

2. Bioprothèse selon la revendication 1, caractérisée en ce que ledit support (2) comprend une base annulaire (A) et une superstructure (B) avec trois bras (d) disposés à 120° sur ladite base (A) et reliés selon une courbe (f) semblable à une valvule biologique naturelle, chaque bras (d) présentant une découpe sensiblement en triangle isocèle, chacune desdites découpes présentant un petit côté (g) sur la base (A) et son sommet qui est opposé à la base (A) étant muni d'une protubérance (i) pour fixer le matériau de la couture.

3. Procédé de fabrication d'une bioprothèse valvulaire cardiaque selon la revendication 1 ou 2, caractérisé en ce qu'une valvule aortique, une paroi aortique et une partie du septum interventriculaire sont prélevés immédiatement après l'abattage d'un animal, lavés au moyen d'un sérum physiologique pour en éliminer le sang, et en ce que la valvule est appliquée sur un support métallique ou plastique, préalablement revêtu d'un élément en tissu et muni d'une bague sur son côté externe, caractérisé par les étapes suivantes:

le prélèvement de ladite valvule aortique, de la paroi aortique et de ladite partie du septum ventriculaire d'un sanglier ou d'un ours,

l'élimination des fibres musculaires des cuspides septales et du surplus du tissue de l'aorte,

l'introduction de tampons dans les cuspides, qui sont ensuite introduites dans une solution de conservation et de stérilisation dans des récipients à 5—12°C,

le retrait du matériau biologique après 48 heures et le pelage de la paroi aortique,

le revêtement séparé dudit support complètement au moyen d'une matière tricotée cylindrique en fixant un cylindre extérieur de celle-ci au support au moyen de l'application de ladite bague, qui est en une matière textile, une matière plastique ou en caoutchouc au silicone, et en pliant et en cousant un cylindre intérieur de celle-ci par dessus la bague,

la fixation du matériau biologique préparé au moyen de deux coutures sur le support, et

l'application d'une bande de matériau tricoté en vue de la protection de la couture.

## Patentansprüche

1. Biologische Herzklappenprothese, bestehend aus einer Aortenklappe aus biologischem Material (1), das auf einem Metall- oder Kunststoffträger (2) mit einer Verbindungsschicht (3) und einem Befestigungsring (4) angebracht ist, gekennzeichnet durch eine Aortenklappe von einem Wildschwein oder einem Bär, und einer Verbindungsschicht (3) aus gewirktem Textilmaterial, wobei das biologische Material einen inneren zylindrischen Bestandteil und einen äußeren zylindrischen Bestandteil (1) aufweist, der an dem Träger (2) mittels des Rings (4) befestigt ist, welcher aus einem Textilmaterial, einem Kunststoffmaterial oder aus einem Siliconkautschuk besteht.

2. Biologische Prothese nach Anspruch 1, dadurch gekennzeichnet, daß der Träger (2) mit einem ringförmigen Grundteil (A) und einem Überbau (B) mit drei Armen (D) gebildet ist, die unter einem Winkel von 120° auf dem Grundteil (A) angeordnet und über eine Krümmung (f) ähnlich der natürlichen biologischen Klappe miteinander verbunden sind, wobei jeder Arm (D) einen im wesentlichen gleichschenkligen dreieckigen Ausschnitt besitzt, wobei jeder von den Ausschnitten eine kleine Seite (g) auf dem Grundteil (A) besitzt und wobei seine Spitze gegenüber dem Grundteil (A) mit einem vorstehenden Ansatz (i) zur Befestigung des Saummaterials versehen ist.

3. Verfahren zur Herstellung der biologischen Herzklappenprothese gemäß Anspruch 1 oder 2, bei dem eine Aortenklappe, eine Aortenwand und ein Teil des interventrikulären Septums unmittelbar nach dem Schlachten eines Tiers entnommen werden, mit einem physiologischem Serum zum Entfernen von Blut gewaschen werden und bei dem die Klappe auf einen Metall- oder Kunststoffträger aufgebracht wird, der zuvor mit einem Gewebeteil überzogen und mit einem ring an der Außenseite versehen worden ist, gekennzeichnet durch die folgenden Verfahrensschritte:

Entnahme der Aortenklappe, der Aortenwand und des Teils des ventrikularen Septums von einem Wildschwein oder einem Bär;

Entfernen von Muskelfasern von dem Septumzipfeln und von überschüssigem Gewebe von der Aorta;

Einführen von Austauschteilen in die Zipfel, welche danach in eine konservierende und sterilisierende Lösung in Behälter bei einer Temperatur von 5—12°C eingeführt werden;

Entnahme des biologischen Materials nach 48 Stunden und Abschälen der Aortenwand;

Separates vollständiges Beziehen des Trägers mit einem zylindrischen gewirkten Material, indem ein Außenzylinder desselben an dem Träger mittels des Anbringens des Ringes befestigt wird, der aus einem Textilmaterial, einem Kunststoffmaterial oder Siliconkautschuk besteht, und indem ein innerer Zylinder desselben über den Ring gebogen und genäht wird;

Befestigen des präparierten biologischen Materials mit zwei Nähten an dem Träger; und

Aufbringen eines Bandes aus gewirktem Material zum Schutz der Naht.

FIG. 1

A-A

B-B

FIG. 2.

FIG. 3

FIG. 4